# EUROPEAN PATENT SPECIFICATION

(11) **EP 1 123 509 B1**
(45) Date of publication and mention of the grant of the patent: **29.12.2004**
(21) Application number: 99971091.6
(22) Date of filing: 22.10.1999
(51) Int. Cl.: G01N 33/68

(54) **METHODS OF DIAGNOSING OR PROGNOSING ALZHEIMER'S DISEASE**
METHODEN ZUR DIAGNOSE ODER PROGNOSE DER ALZHEIMERSCHEN KRANKHEIT
METHODES DE DIAGNOSTIC OU DE PRONOSTIC DE LA MALADIE D'ALZHEIMER

(30) Priority: 23.10.1998 US 105458 P; 06.11.1998 US 107434 P; 26.01.1999 EP 99101377
(43) Date of publication of application: 16.08.2001
(73) Proprietor: EVOTEC Neurosciences GmbH, 22525 Hamburg (DE)
(72) Inventor: NITSCH, Roger, CH-8702 Zollikon (CH); GROWDON, John, Chestnuthill, MA 02167 (US)
(74) Representative: Meyers, Hans-Wilhelm, Dr.Dipl.-Chem.
(86) International application number: PCT/EP1999/008023
(87) International publication number: WO 2000/025138

(56) References cited:
- DE-A- 2 711 201
- FUJIHARA S ET AL: "CEREBRAL AMYLOID ANGIOPATHY WITH THE DEPOSITION OF CYSTATIN C GAMMA-TRACE AND BETA-PROTEIN." KAMEYAMA, M. (ED.). INTERNATIONAL CONGRESS SERIES, NO. 999. BETA-AMYLOID PRECURSOR PROTEINS AND NEUROTRANSMITTER FUNCTION;EIGHTH WORKSHOP ON NEUROTRANSMITTERS AND DISEASES, TOKYO, JAPAN, JUNE 1, 1991. VII+107P. ELSEVIER SCIENCE PUBLISHERS B.V.: AMSTE, XP002110418 cited in the application
- VINTERS, H.V. ET AL.: "Immunoreactive A4 and Gamma-trace Peptide Colocalization in Amyloidotic Arteriolar Lesions in Brains of Patients with Alzheimer's Disease" THE AMERICAN JOURNAL OF PATHOLOGY, vol. 137, no. 2, August 1990 (1990-08), pages 233-240, XP000866515
- MARUYAMA, KEIKO ET AL.: "Immunohistochemical Characterization of Cerebrovascular Amyloid in 46 Autopsied Cases Using Antibodies to beta-Protein and Cystatin C" STROKE, vol. 21, no. 3, March 1990 (1990-03), pages 397-403, XP000869870
- PETURSDOTTIR I ET AL: "ESTIMATION OF CYSTATIN C IN DIFFERENT HUMAN EXTRACELLULAR FLUIDS." FIRST SYMPOSIUM ON HEREDITARY CENTRAL NERVOUS SYSTEM AMYLOID ANGIOPATHY, REYKJAVIK, ICELAND, SEPT. 2-3, 1985. ACTA NEUROL SCAND. (1986) 73 (3), 318., XP002110416 cited in the application
- LÖFBERG, H. ET AL.: "The Cerebrospinal Fluid and Plasma Concentrations of Gamma-Trace and Beta-2-Microglobulin at Various Ages and in Neurological Disorders" JOURNAL OF NEUROLOGY, vol. 223, no. 3, 1980, pages 159-170, XP002110417 cited in the application
- Olafsson et al: Scand. J. Lab. Invest vol. 48, 1988, p. 573-582
- Davidsson et al: J. Neural Transmission, vol. 104, No. 6-7, 1997. p. 711-720

## Description

Cerebral amyloid angiopathies comprise a heterogeneous group of disorders that are characterized clinically by ischaemic and/or haemorrhagic strokes, and histologically by deposition of amyloid in the wall of leptomeningeal and cerebral cortical blood vessels. Two forms can be distinguished on the basis of the molecular composition of the amyloid: (i) cystatin C amyloid angiopathy is a rare autosomal dominant disorder confined to several families from Iceland and (ii) β-amyloid cerebral amyloid angiopathies may be hereditary or sporadic, and share clinical, pathological and biochemical features with Alzheimer's disease (Coria et al., Neuropathology and applied neurobiology, 22(3), 216 - 227, 1996).

Alzheimer's disease (AD), first described by the Bavarian psychiatrist Alois Alzheimer in 1907, is a progressive neurological disorder which begins with short term memory loss and proceeds to loss of cognitive functions, disorientation, impairment of judgment and reasoning and, ultimately, dementia. It is the most common cause of dementia. The neuropathology is characterized by the formation in brain of amyloid plaques and neurofibrillary tangles. AD has been estimated to afflict 5 to 11 percent of the population over age 65 and as much as 47 percent of the population over age 85. Moreover, as adults, born during the population boom of the 1940's and 1950's, approach the age when AD becomes more prevalent, the control and treatment of AD will become an even more significant health care problem. Familial forms of AD are genetically heterogeneous, but most with early onset are linked to mutations in the presenilin genes *PSEN1* and *PSEN2*, as well as to mutations of the amyloid precursor gene *APP*. The majority of AD patients have no obvious family history and are classified as sporadic AD.

In the search for biochemical changes in patients with neurological disorders such as AD, analysis of cerebrospinal fluid (CSF) may be a useful method, since the CSF is continuous with the extracellular fluid of the brain. Therefore, a plurality of studies aiming at the analysis of central nervous system (CNS) specific proteins in CSF were performed in order to find biochemical markers for neuronal and synaptic function and pathology in degenerative brain disorders. Further studies were directed to the identification of possible genetic risk factors including polymorphisms in the human cystatin C gene. The human cystatin C gene (*CST3*) maps to chromosome 20p11.2 and contains three exons. Four point mutations in the promoter region of the human cystatin C gene have been detected by direct sequencing of polymerase chain reaction amplified D N A. The four base changes are all localized within a short segment of 85 base pairs. Three cystatin C gene alleles could be defined with respect to these promoter mutations. Mendelian inheritance of the polymorphisms has been demonstrated in a study of Caucasian individuals showing frequencies of cystatin C genotypes AA, BB, CC, AB, AC and BC. An Ala/Thr variation in the coding region of the human cystatin C gene has been detected as a *Sst* II polymorphism (Balbin et al., Biol.Chem. Hoppe-Seyler, Vol. 373, pp. 471 - 476, July 1992; Balbin and Abrahamson, Hum. Genet. 81, 751 - 752, 1991; Balbin et al., Hum. Genet. 92, 206 - 207, 1993; Abrahamson et al., FEBS Lett. 216, 229 - 233, 1987). The open reading frame of *CST3* encodes a 120-residue protein with a molecular mass of 13.3 kDa and a pl of 8.75 (Abrahamson et al., J. Biol. Chem. 261, 11282 - 11289, 1986; Abrahamson et al., FEBS Lett. 216, 229 - 233, 1987). The mature molecule contains intramolecular disulfide bonds, it is partially hydroxylated, no other common post-translational modifications were observed (Grubb and Löfberg, Proc. Natl. Acad. Sci., USA, 79, 3024 - 3027, 1982; Asgeirsson et al., Biochem. J., 329, 497 - 503, 1998). Cystatin C is distributed extensively in the body fluids and is suspected of playing a role in extracellular functions, such as the modulation of inflammatory reactions. It is known to exist in cell types, such as astrocytes, macrophages, and choroid plexus cells. Cystatin C also is a quantitatively dominating cysteine protease inhibitor of CSF whose concentration is five times higher than that of plasma. It binds to and regulates proteolytic activities of cathepsins which have been associated with brain amyloid plaques in Alzheimer's disease, and implicated in the proteolytic processing of the amyloid precursor protein. It has been described that human cystatin C undergoes dimerization before unfolding. Dimerization leads to a complete loss of its activity as a cysteine proteinase inhibitor (Ekiel et al., J. Mol. Biol. 271, 266 - 277, 1997). In addition to the termination of biological activity, dimerization of cystatin C is associated with brain amyloid formation in the Islandic form of hereditary cerebral hemorrhage with amyloidosis caused by an inherited mutation (L68Q) within the coding region of *CST3*.

The production rate of cystatin C is remarkably constant and its plasma conçentration can therefore be used as a reliable measure of the glomerular filtration rate (Grubb, Clinical Nephrology, Vol. 38, Suppl. No.1, 20 - 27, 1992).

Nagai et al. (Molecular and Chemical Neuropathology, Vol. 33, p. 63 - 78, 1998) found that cerebral amyloid angiopathy (CAA) patients, on whose cerebral blood vessels cystatin C colocalized with β-protein, had low concentration of cystatin C in their cerebrospinal fluid (CSF). DNA analysis of these patients showed no point mutations in the cystatin C gene in contrast to hereditary cerebral hemorrhage with amyloidosis, lcelandic type (HCHWA-I) characterized by a variant cystatin C with an amino acid substitution at position 68 and a corresponding point mutation at the genetic level. An abnormally low level of cystatin C in CSF as a diagnostic marker for the hereditary form of brain hemorrhage associated with amyloidosis in Iceland was confirmed by Shimode and co-workers who developed an assay for cystatin C to be used in the diagnosis of patients with cerebral amyloid angiopathy and brain hemorrhage (Stroke, Vol. 22, 860 - 866, 1991).

Petursdottir et al. (First symposium on hereditary central nervous system amyloid angiophathy, Reykjavik, Iceland, Sep. 2 -3 , 1985. Acta Neurol. Scand. 73(3), 318, XP002110416, 1986) measured cystatin C in the cerebrospinal fluid of adult Down's syndrome individuals.

Fujihara et al. (Kameyama (ed.). International congress series, No. 999. Betaamyloid precursor proteins and neurotransmitter function; eigth workshop on neurotransmitters and diseases, Tokyo, Japan, June 1, 1991. VII+107P. Elsevier Science Publishers B.V. Amste, XP002110418) found in Japanese cases of amyloid angiopathy an antigenicity of cystatin C as well as of β-protein within amyloid plaques. Patients with amyloid angiopathy with the deposition of cystatin C had low concentration of cystatin C in cerebrospinal fluid.

The use of determinations of the CSF concentration of cystatin C - also termed γ-trace - in the diagnosis of hereditary cerebral hemorrhage with γ-trace-amyloidosis was described by Grubb and Löfberg (Scand. J. Clin. Lab. Invest., 45, Suppl. 177: 7 - 13, 1985).

Shimode et al. (Stroke, Vol. 27, 1417 - 1419, 1996) further found out that measurement of cystatin C in CSF by enzyme-linked immunosorbent assay (ELISA) is a useful method of diagnosing leukoencephalopathy related to sporadic cystatin-C type cerebral amyloid angiopathy.

Regarding HCHWA-I, identification of the disease-causing mutation and specific diagnosis by polymerase chain reaction based analysis has been described by Abrahamson et al. (Hum. Genet. 89: 377 - 380, 1992).

Davidsson et al. (J. Neural. Transm., 104: 711 - 720, 1997) describe a procedure for detecting brain-specific proteins in cerebrospinal fluid. After three affinity chromatography steps, intended to remove interfering serum proteins from CSF, micro-reversed HPLC revealed four major peaks, which by both Western blotting and mass spectrometric analyses were found to correspond to β2-micro-globulin, cystatin C, transthyretin (TTR) and asialotransferrin. When comparing these peaks in CSF from Alzheimer's disease patients and age-matched healthy controls, only a reduction of the brain-specific pl 5.7 form of TTR and an increase in the pl 5.4 form of TTR was found.

Löfberg et al. (J. Neurol. 223, 159 - 170, 1980) disclose the existence of a connection between the cerebrospinal fluid (CSF) and plasma level of γ-trace and the age of an individual. These results emphasize the necessity of using age-matched reference values when CSF and plasma levels of γ-trace are to be evaluated in different groups of patients. Children and adults with well defined neurological disorders were also examined in regard to their levels of y-trace. These children suffered from acute aseptic meningitis; or meningococcal meningitis; or group B streptococcal septicemia and meningitis; or meningeal lymphoma; or infantile myoclonic seizures; or later developed cerebral palsy syndromes. The adults suffered from multiple sclerosis; or cerebral infarctions; or transitory ischemic attack; or reversible ischemic neurological deficit; or intracerebral, subdural or subarachnoid hemorrhage; or cerebral atrophy; or acoustic neuroma; or intramedullary gliomas; or intraspinal neuroma, or meningeal carcinomatosis; or Hodgkin's disease; or meningitis; or encephalitis; or chorioretinitis; or myelitis; or systemic lupus erythematosus; or arteritis with cerebral symptoms; or Guillain-Barré syndrome; or epilepsy; or polyneuropathy; or intervertebral disc protusion. Significantly increased CSF levels of γ-trace and increased plasma concentration of γ-trace were found in infectious disorders, i.e. meningitis, encephalitis, chorioretinitis and myelitis. Increased γ-trace concentration in plasma were seen in the above mentioned cerebrovascular disorders, i.e. cerebral infarction, transitory ischemic attack, reversible ischemic neurological deficit, intracerebral, subdural or subarachnoid hemorrhage, as well as in infectious disorders. Löfberg did not examine any patients suffering from Alzheimer's disease.

As Alzheimer's disease is a growing social and medical problem, there is a strong need for methods of diagnosing or prognosing said disease in subjects as well as for methods of treatment. In addition, there is a strong need for identifying inherited risk factors that increase the susceptibility of getting AD (susceptibility gene).

In one aspect, the invention features a method for diagnosing or prognosing Alzheimer's disease in a subject, or determining whether a subject is at increased risk of developing Alzheimer's disease according to claim 1.

In a further aspect, the invention features a method of monitoring the progression of Alzheimer's disease in a subject according to claim 2.

In still a further aspect, the invention features a method of evaluating a treatment for Alzheimer's disease according to claim 3.

Preferred embodiments of the above mentioned methods for diagnosing or prognosing AD, or determining an increased risk of becoming diseased with AD, or monitoring the progression of AD, or evaluating a treatment of Alzheimer's disease are now disclosed in detail.

It might be preferred that a subject has previously been determined to have one or more factors indicating that such subject is afflicted with Alzheimer's disease.

The sample is a cerebrospinal fluid. According to the present invention, an increase of a level or a varied activity of a translation product of (i) a cystatin C gene or (ii) a polymorphic variant of a cystatin C gene in cerebrospinal fluid from the subject relative to a reference value representing a known health status indicates a diagnosis, or prognosis, or increased risk of Alzheimer's disease in said subject. In particular, an increase of a level of cystatin C in cerebrospinal fluid compared to control subjects is an indicator for Alzheimer's disease. Furthermore, the appearance of a translation product of a polymorphic variant of a cystatin C gene is an indicator for Alzheimer's disease. In case of polymorphisms in the coding region of the gene, this translation product has an amino acid sequence which differs from that of cystatin C in its wild-type form. Usually, a control subject with a wild-type cystatin C gene will be chosen. In this control subject, one will therefore determine only wild-type cystatin C. Accordingly, the appearance of a translation product of a polymorphic variant of a cystatin C gene in a patient in comparison to a reference value of preferably a wild-type control subject shall be within the scope of the term "increase of a level of a translation product of a polymorphic variant of a cystatin C gene". However, polymorphisms might also be extant in regions of ordinary skill in the art (see Harlow et al., Antibodies, A Laboratory Manual, Cold Spring Harbor Laboratory Press, Cold Spring Harbor, New York).

The antibody or ligand to be used should preferably specifically detect a translation product of (i) a cystatin C gene or (ii) a polymorphic variant of a cystatin C gene. It is preferred that it does not interact with any other protein present in said sample. It is particularly preferred to include specific antibodies or ligands which differentiate monomers from dimers or oligomers of a translation product of (i) a cystatin C gene or (ii) a polymorphic variant of a cystatin C gene. As shown in Example 1, the detection of the monomer form of cystatin C is more specific to AD than measuring dimers or oligomers. Measurements are significantly improved with monomer-specific ELISAs.

Monoclonal antibodies capable of recognizing a translation product of (i) a cystatin C gene or (ii) a polymorphic variant of a cystatin C gene can be prepared using methods known in the art (see e.g. Köhler and Milstein, Nature 256, 495 - 497 1975; Kozbor et al., Immunol. Today 4, 72, 1983; Cole et al., Monoclonal antibodies and cancer therapy, Alan R. Liss, Inc., pp 77 - 96, 1985; Marks et al., J. Biol. Chem., 16007 - 16010, 1992). Such monoclonal antibodies or fragments thereof can also be produced by alternative methods known to those of skill in the art of recombinant DNA technology (see e.g. Sastry et al, PNAS 86: 5728, 1989; Watson et al., Rekombinierte DNA, 2nd ed., Spektrum Akademischer Verlag GmbH, 1993; Watson et al, Recombinant DNA, 2nd ed., W. H. Freeman and Company, 1992). Monoclonal antibodies useful in the methods of the invention are directed to an epitope of a translation product of (i) a cystatin C gene or (ii) a polymorphic variant of a cystatin C gene, such that the complex formed between the antibody and said translation product can be recognized in detection assays. The term "antibodies" encompasses all forms of antibodies known in the art, such as polyclonal, monoclonal, chimeric, recombinatorial, single chain antibodies as well as fragments thereof which specifically bind to a translation product of (i) a cystatin C gene or (ii) a polymorphic variant of a cystatin C gene. It is particularly preferred to use specific antibodies that selectively detect monomers, dimers or oligomers, respectively, of cystatin C or said translation product of a polymorphic variant of a cystatin C gene. High-affinity ligands can be prepared by using derivatives of cathepsins which are the natural substrates of cystatin C biological activity.

It is further preferred to determine the level and/or activity of a translation product of (i) a cystatin C gene or (ii) a polymorphic variant of a cystatin C gene on basis of an enzymatic assay. As described above, cystatin C is a cysteine protease inhibitor which binds to and regulates proteolytic activities of cathepsins. A suitable enzymatic assay can therefore be built upon the enzymatic activity of cathepsins indicating the absense or presence of different levels of cystatin C or a translation product of a polymorphic variant of a cystatin C gene in its active monomer form. It is preferred to use amyloid precursor protein (APP) as a substrate. The generation of A-beta peptides as products can be measured.

The determination of a level and/or an activity of a translation product of (i) a cystatin C gene or (ii) a polymorphic variant of a cystatin C gene can also be performed on basis of a binding assay. Suitable binding partners include cathepsins or fragments thereof, peptides, peptidomimetics, antibodies and other chemical probes which can specifically recognize a translation product of (i) a cystatin C gene or (ii) a polymorphic variant thereof. It is in general particularly preferred to determine said translation products in their monomer form.

If luminescent labels are used in any detection assay, it is preferred to use a confocal optical set-up.

In preferred embodiments, the reference value can be that of a level, or an activity, or both said level and said activity, of a transcription product and/or a translation product of (i) a cystatin C gene or (ii) a polymorphic variant of a cystatin C gene in a sample, preferably a body fluid, from a subject not suffering from Alzheimer's disease. The healthy subject can be of the same weight, age, and gender as the subject who is being diagnosed or prognosed for Alzheimer's disease, or for whom an increased risk of becoming diseased with Alzheimer's disease is determined. In some cases, it might be preferred to use a reference value from the subject which is diagnosed.

In preferred embodiments, the subject can be a human, an experimental animal, e.g. a rat or a mouse, a domestic animal, or a non-human primate, e.g. a monkey. The experimental animal can be an animal model for a disorder, e.g. a transgenic mouse with an Alzheimer's-type neuropathology.

It is preferred to determine a level, or an activity, or both said level and said activity of a transcription product and/or a translation product of (i) a cystatin C gene or (ii) a polymorphic variant of a cystatin C gene at least twice, e.g. at two points which are weeks or months apart. The levels or activities at these two time points are compared in order to monitor the progression of Alzheimer's disease. It might further be preferred to compare a level, or an activity, or both said level and said activity of a transcription product and/or a translation product of (i) a cystatin C gene or (ii) a polymorphic variant of a cystatin C gene in said sample with a level, or an activity, or both said level and said activity, of at least one of said products in a series of samples taken from said subject over a period of time. In further preferred embodiments, said subject receives a treatment prior to one or more of said sample gatherings.

In another aspect, the invention features, a method of diagnosing or prognosing Alzheimer's disease in a subject, or determining whether a subject is at increased risk of becoming diseased with Alzheimer's disease. The method includes: determining the presence or absense of a polymorphism in a cystatin C gene in a sample from said subject, thereby diagnosing, or prognosing AD in said subject, or determining whether said subject is at increased risk of developing Alzheimer's disease. Polymorphisms and allele variations occur more frequently in a population than random mutations but induce in principle the identical genetic alterations. A mutation can e.g. be a substitution, deletion or addition of at least one base. Such mutations may result in "mis-sense" information or in "non-sense" information associated with a termination codon or a frame shift. Polymorphisms may be found within the promoter region, an example of which is the *Sst* II polymorphic site in the promoter region of the human cystatin C gene (*CST 3*). Polymorphisms may also be found within coding regions of a gene. An Ala/Thr variation in the coding region of the human cystatin C gene has been detected as a *Sst* II polymorphism. With respect to the human cystatin C gene, it is preferred to determine the presence of a B allele. The human cystatin C gene, called *CST3*, has been sequenced and its A, B and C alleles have been described (Balbin et al., Biol. Chem. Hoppe-Seyler, Vol. 373, 471 - 476, 1992; Abrahamson et al., Biochem. J. 268, 287 - 294, 1990; Abrahamson et al., Hum. Genet. 82, 223 - 226, 1989). The presence of at least one B allele indicates said subject and potentially its descendants are at increased risk of developing Alzheimer's disease. In particular, homozygous *CST 3* B/B subjects are at increased risk of developing Alzheimer's disease. The data shown in Example 2 indicate an association of *CST3* B/B genotype with AD, and they suggest that inheritance of the *CST3* B/B genotype is a risk factor for AD, with estimated odds ratios of 5.34 to 10.7, but without modifying the age of onset. Thus, the *CST3* B/B genotype is a stronger risk factor for AD than inheritance of *ApoE* ε4 alleles or inheritance of the G/G or *A2M-2* alleles of the alpha-2 macroglobulin gene with odds ratios between 1.77 and 3.56.

Determining the presence or absense of polymorphism in a cystatin C gene in a sample from said subject may comprise determining a partial nucleotide sequence of the DNA from said subject, said partial nucleotide sequence indicating the presence or absence of said polymorphism. It may further be preferred to perform a polymerase chain reaction with the DNA from said subject and subsequent restriction analysis to determine the presence or absence of a polymorphism. Such techniques are known to those of ordinary skill in the art (see Lewin, B., Genes V, Oxford University Press, 1994). In a further preferred embodiment, primers depicted in SEQ ID NO. 3 and SEQ ID NO. 4 are used for amplifying parts of the promoter region as well as the coding sequence of exon 1 of the human cystatin C gene in order to subsequently analyze the *Sst* II polymorphic sites herein.

A polymorphism can be found in leucin 68 codon of a human cystatin C gene which leads to a loss of an *Alu* I restriction site. Such a polymorphism does not indicate diagnosis, prognosis or increased risk of Alzheimer's disease. It is rather indicative for the Islandic form of hereditary cerebral hemorrhage with amyloidosis Shimode et al, supra.

In another preferred embodiment, the method further comprises: determining a level, or an activity, or both said level and said activity, of a transcription product and/or a translation product of (i) a cystatin C gene or (ii) a polymorphic variant of a cystatin C gene in a sample from said subject; and comparing said level, or said activity, or both said level and said activity, of said transcription product and/or said translation product to a reference value. Preferred embodiments of this method are described in detail above. The association of the cystatin C genotype with the phenotype of elevated CSF levels as shown in Table 5 and Example 2 underscores the role of cystatin C in the pathophysiology of AD. Increased CSF levels of cystatin C seem to confer the increased risk for AD associated with at least one B allele, especially with the homozygous "BB" cystatin C genotype, e.g. *CST3* B/B. The lowest CSF levels of cystatin C were found in healthy *CST3* A carriers.

In preferred embodiments, the sample comprises tissues, cells like white blood cells or skin fibroblasts, or body fluids like e.g. cerebrospinal fluid, saliva, urine or serum plasma. For the determination of polymorphisms in the cystatin C gene, it is preferred to use DNA from body cells, in particular white blood cells.

In another aspect, the invention features a kit for diagnosis, or prognosis, or determination of increased risk of Alzheimer's disease in a subject.
Said kit comprises:
- reagents that selectively detect the presence or absence of the B allele of the cystatin C gene.

The kit may further comprise reagents that selectively detect a transcription product of (i) a cystatin C gene or (ii) a polymorphic variant of a cystatin C gene and/or reagents that selectively detect a translation product of (i) a cystatin gene or (ii) a polymorphic variant of a cystatin C gene.

It is preferred that said at least one reagent and said instructions are packaged in a single container. In preferred embodiments, said reference value is that of a level, or an activity, or both said level and said activity, of a transcription product and/or a translation product of (i) a cystatin C gene or (ii) a polymorphic variant of a cystatin C gene in a sample from a subject not suffering from said Alzheimer's disease. The healthy subject can be of the same weight, age, and gender as the subject who is being diagnosed, or prognosed for Alzheimer's disease, or for whom an increased risk of developing Alzeheimer's disease is determined. In some cases, it might be preferred to use a reference value of the subject which is to be diagnosed. Said kit suitable for commercial manufacture and sale can still further include appropriate standards, positive and negative controls. It is preferred that said kit further comprises reagents to assess a function or dysfunction of said subject's kidneys.

In further preferred embodiments, the presence of at least one B allele, in particular the presence of the B/B genotype of the cystatin C gene, indicates a diagnosis, or prognosis, or an increased risk of Alzheimer's disease. In order to exclude a false positive diagnosis, it should be remarked that a mutation or polymorphism in the cystatin C gene in the codon for leucine at position 68 which abolishes an *Alu*I restriction site is in principle not indicative for Alzheimer's disease, but for hereditary cystatin C amyloid angiopathy.

Determining the presence or absense of a mutation or polymorphism in a cystatin C gene in a sample from said subject may comprise determining a partial nucleotide sequence of the DNA from said subject, said partial nucleotide sequence indicating the presence or absence of said mutation or polymorphism. It may further be preferred to perform a polymerase chain reaction with the DNA from said subject and subsequent restriction analysis to determine the presence or absence of said mutation or polymorphism. Such techniques are known to those of ordinary skill in the art (see Lewin, B., Genes V, Oxford University Press, 1994). In a further preferred embodiment, primers depicted in SEQ ID NO. 3 and SEQ ID NO. 4 are used for amplifying parts of the promoter region as well as the coding sequence of exon 1 of the human cystatin C gene in order to subsequently analyze the *Sst* II polymorphic sites herein.

In preferred embodiments, measurement of the level of transcription products of (i) a cystatin C gene or (ii) a polymorphic variant of a cystatin C gene is performed in body cells using Northern blots with probes specific for the cystatin C gene or said variant. Quantitative PCR with primer combinations to amplify sequences from cDNA obtained by reverse transcription of RNA extracted from body cells of a subject can also be applied. These techniques are known to those of ordinary skill in the art (see e.g. Watson et al., Rekombinierte DNA, 2nd edition, Spektrum Akademischer Verlag GmbH, Heidelberg, 1993; Watson et al., Recombinant DNA, 2nd ed., W. H. Freeman and Company, 1992).

In preferred embodiments, the sample is a body fluid like e.g. cerebrospinal fluid, saliva, urine or serum plasma, or a tissue, or cells like blood cells or skin fibroblasts. For the determination of mutations or polymorphisms in the cystatin C gene, it is preferred to use DNA from body cells including fibroblasts.and white blood cells. For the other analyses, it is particularly preferred to use cerebrospinal fluid. According to the present invention, an increase of a level or a varied activity of a translation product of (i) a cystatin C gene or (ii) a polymorphic variant of a cystatin C gene in cerebrospinal fluid from the subject relative to a reference value representing a known health status indicates a diagnosis, or prognosis, or increased risk of said Alzheimer's disease in said subject. In particular, an increase of a level of cystatin C in cerebrospinal fluid compared to control subjects is an indicator for Alzheimer's disease.

Furthermore, the appearance of a translation product of a polymorphic variant of a cystatin C gene is an indicator for Alzheimer's disease. In case of polymorphisms in the coding region of the gene, this translation product has an amino acid sequence which differs from that of cystatin C in its wild-type form. Usually, a control subject with a wild-type cystatin C gene will be chosen. In this control subject, one will therefore determine only wild-type cystatin C. Accordingly, the appearance of a translation product of a polymorphic variant of a cystatin C gene in a patient in comparison to a reference value of preferably a wild-type control subject shall be within the scope of the term "increase of a level of a translation product of a polymorphic variant of a cystatin C gene". However, polymorphisms might also be extant in regions preceding and/or following the coding region (leader and trailer) or in intervening sequences (introns) between individual coding segments (exons). As shown in Example 1, CSF levels of cystatin C, in particular in its monomer form (which is the one with biological activity), are significantly elevated in AD as compared to normal control subjects or to patients with non-AD neurological diseases, indicating the important role of cystatin C for the early diagnosis of AD. Because CSF levels of cystatin C measured by both ELISA and Western blotting were significantly lower in both non-AD neurodegenerative and infectious brain diseases, compared to AD, non-specific effects of neurodegeneration or inflammation on CSF levels of cystatin C are highly unlikely (see Example 1).

In preferred embodiments, said level and/or activity of a translation product of (i) a cystatin C gene or (ii) a polymorphic variant of a cystatin C gene is detected using an immunoassay. These assays can e.g. measure the amount of binding between cystatin C and an anti-cystatin C antibody by the use of enzymatic, chromodynamic, radioactive, or luminescent labels which are attached to either the anti-cystatin C antibody or a secondary antibody which binds the anti-cystatin C antibody. In addition, other high affinity ligands including cathepsin derivatives may be used. Immunoassays which can be used include e.g. ELISAs, Western blots and other techniques known to those of ordinary skill in the art (see Harlow et al., Antibodies, A Laboratory Manual, Cold Spring Harbor Laboratory Press, Cold Spring Harbor, New York).

The antibody or ligand to be used should preferably specifically detect a translation product of (i) a cystatin C gene or (ii) a polymorphic variant of a cystatin C gene. It is preferred that it does not substantially interact with any other protein present in said sample. It is particularly preferred to include specific antibodies or ligands which differentiate monomers from dimers or oligomers of said translation products. As shown in Example 1, the detection of the monomer form of cystatin C is more specific to AD than measuring dimers or oligomers. Measurements might be significantly improved with monomer-specific ELISAs.

Monoclonal antibodies capable of recognizing a translation product of (i) a cystatin C gene or (ii) a polymorphic variant of a cystatin C gene can be prepared using methods known in the art (see e.g. Köhler and Milstein, Nature 256, 495 - 497 1975; Kozbor et al., Immunol. Today 4, 72, 1983; Cole et al., Monoclonal antibodies and cancer therapy, Alan R. Liss, Inc., pp 77 - 96, 1985; Marks et al., J. Biol. Chem., 16007 - 16010, 1992). Such monoclonal antibodies or fragments thereof can also be produced by alternative methods known to those of skill in the art of recombinant DNA technology (see e.g. Sastry et al, PNAS 86: 5728, 1989; Watson et al., Rekombinierte DNA, 2nd ed., Spektrum Akademischer Verlag GmbH, 1993; Watson et al., Recombinant DNA, 2nd ed., W. H. Freeman and Company, 1992). Monoclonal antibodies useful in the methods of the invention are directed to an epitope of a translation product of (i) a cystatin C gene or (ii) a polymorphic variant of a cystatin C gene, such that the complex formed between the antibody and said translation product can be recognized in detection assays. The term "antibodies" encompasses all forms of antibodies known in the art, such as polyclonal, monoclonal, chimeric, recombinatorial, single chain antibodies as well as fragments thereof which specifically bind to cystatin C, or to a translation product of a polymorphic variant of a cystatin C gene. It is particularly preferred to use specific antibodies that selectively detect monomers, dimers or oligomers, respectively, of a translation product of (i) a cystatin C gene or (ii) a polymorphic variant of a cystatin C gene. High-affinity ligands can be prepared by using derivatives of cathepsins which are the natural substrates of cystatin C biological activity.

It is further preferred to determine a level and/or an activity of a translation product of (i) a cystatin C gene or (ii) a polymorphic variant of a cystatin C gene on basis of an enzymatic assay. As described above, cystatin C is a cysteine protease inhibitor which binds to and regulates proteolytic activities of cathepsins. A suitable enzymatic assay can therefore be built upon the enzymatic activity of cathepsins indicating the absense or presence of different levels of cystatin C in its active, monomer form. It is preferred to use amyloid precursor protein (APP) as a substrate. The generation of A-beta peptides as products can be measured.

The determination of a level and/or an activity of a translation product of (i) a cystatin C gene or (ii) a polymorphic variant of a cystatin C gene can also be performed on basis of a binding assay. Suitable binding partners include cathepsins or fragments thereof, peptides, peptidomimetics, antibodies and other chemical probes which can specifically recognize cystatin C or translation products of a polymorphic variant of a cystatin C gene. It is in general particularly preferred to determine translation products in their monomer form.

If luminescent labels are used in any detection assay, it is preferred to use a confocal optical set-up.

In preferred embodiments, the subject can be a human, an experimental animal, e.g. a rat or a mouse, a domestic animal, or a non-human primate, e.g. a monkey. The experimental animal can be an animal model for a disorder, e.g. a transgenic mouse with an Alzheimer's-type neuropathology.

In preferred embodiments, said kit can also be used in monitoring a progression of Alzheimer's disease in a subject or in monitoring success or failure of a therapeutic treatment of said subject.

Other features and advantages of the invention will be apparent from the following detailed description of the examples, and from the claims.

Table 1 shows CSF levels of cystatin C measured by ELISA or by Western blotting and densitometry in AD-patients, non-AD neurological and healthy controls.

Table 2 shows the data obtained by EUSA measurements of cystatin C in human CSF in various neurological and psychiatric diseases.

Table 3 shows the allele frequency of *CST3* B in AD and controls.

Table 4 shows the odds ratios of allelic variants for AD.

Table 5 shows the influence of the *CST3* genotype on CSF levels of cystatin C.

Table 6 shows that there is no effect of *ApoE* genotype on CSF levels of cystatin C.

Figure 1 depicts the correlation of ELISA and Western blotting assays.

Figure 2 depicts the influence of freezing and thawing of CSF samples on their cystatin C level.

Figure 3 depicts a schematic illustration of the human cystatin C gene with the exons numbered and shown as filled boxes as well as the three different alleles (A, B and C) with their respective nucleotide sequences given around the mutations in the promotor region. Nucleotide numbering for the base substitutions relates to the start site for cystatin C translation (+1 - +3 equals initiator methionine codon). The polymorphic *Sst* II and *Dde* I sites are underlined, and expected lengths of DNA fragements after respective cleavage are indicated.

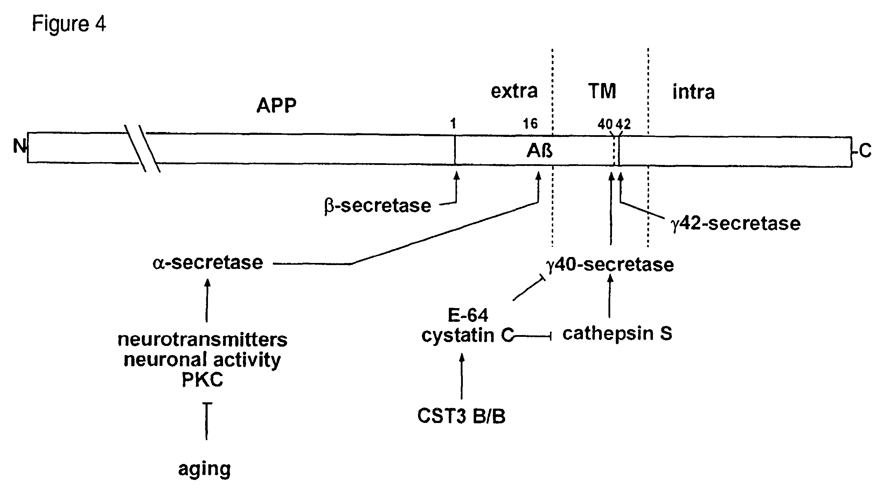

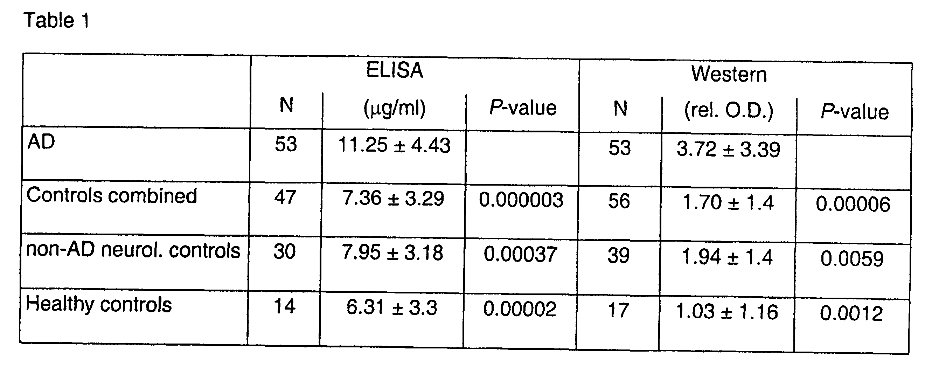

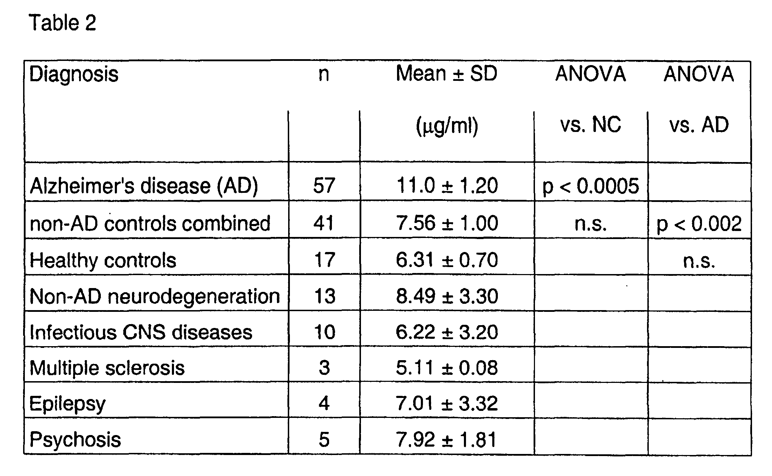

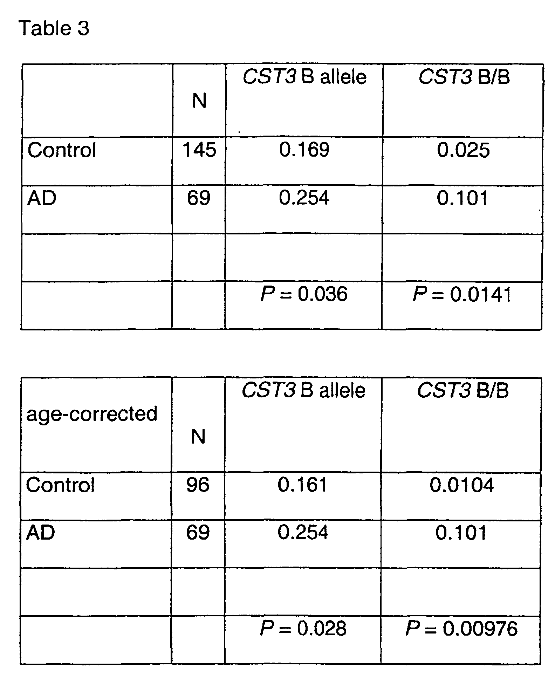

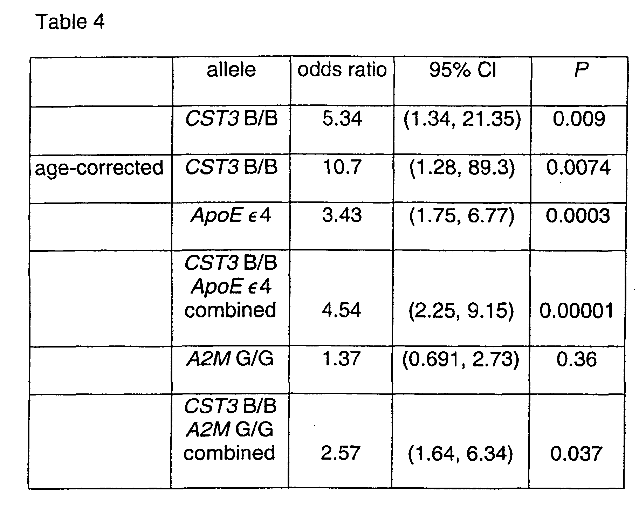

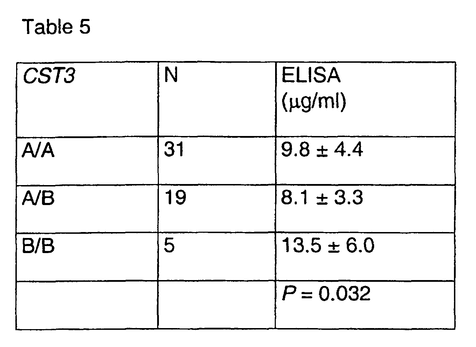

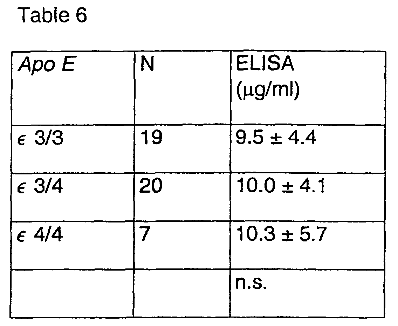

Figure 4 depicts the possible roles of cystatin C and the *Aspergillus japonicus* cysteine proteinase inhibitor E-64 in the amyloid precursor protein (APP) processing and generation of the amyloid β peptide (A_{β}).

### EXAMPLE 1

### Patient characteristics

CSF was collected by lumbar puncture from 56 patients (25 men and 31 women) with a clinical diagnosis of probable AD based on NINDS-ADRDA criteria (McKhann et al., Neurology, 34, 939 - 944, 1984). The mean age of dementia onset was 69.8 ± 8.9 years and the mean age at lumbar puncture was 73.1 ± 8.7 years. At the time of lumbar puncture, the mean score of the Mini Mental State Examination MMSE (Folstein et al., J. Psychiat. Res., 12, 189 - 198, 1975) was 18.1 ± 6.5. Among the AD patients, 18 individuals had an *ApoE* ε3/3 genotype, 27 had *ApoE* ε3/4 heterozygotes, 8 were homozygous for *ApoE* ε4/4, and 2 had an *ApoE* ε2/4 genotype. One AD patient was not genotyped.

There were 38 patients (23 men and 15 women) with neurodegenerative or neurological disorders other than AD that were combined to form a non-AD neurodegenerative disease control (DDC) group. This heterogeneous group included 13 patients with Parkinson's disease, 2 with Huntington's disease, 5 with amyotrophic lateral sclerosis, 5 with epilepsy, and 4 with multiple sclerosis. Other diagnoses in this group included progressive supranuclear palsy, Binswanger's disease, cerebellar atrophy, leukodystrophy, cerebral microangiopathy, and frontal lobe degeneration, HIV-related dementias, borreliosis, alcohol-related dementias, microangiopathy and bipolar psychoses. The mean age of the patients in the DDC group was 56.5 ± 16, and the mean MMSE score was 26 ± 4.7. There were 13 DDC patients with an *ApoE* ε3/3 genotype, 4 with *ApoE* ε3/4, 3 with ApoE ε2/3, and 16 DDC patients were not genotyped.

CSF was also collected from 17 normal control (NC) subjects (11 men and 6 women) who were healthy at the time of lumbar puncture as determined by physical and neurological examination, and who had normal routine blood and CSF tests. Specifically, a dementing illness was excluded clinically and by cognitive testing; the mean MMSE in the normal control group was 29.0 ± 1.2. Of those with a tested *ApoE* genotype, 9 had *ApoE* ε3/3, 3 had *ApoE* ε3/4, 2 had *ApoE* ε2/3. Age was controlled for by two statistical techniques. The first was to stratify the analysis by the decade of lumbar puncture, and the other was to use age as a covatiate in an analysis of covariance. This study protocol was approved by the respective human studies and ethics committees at all participating centers.

### Lumbar puncture

At least three hours before obtaining the CSFs, patients were asked to stay in bed in order to equalize possible gradients of protein concentrations in the spinal canal. CSF was obtained by lumbar puncture with the subject in the lateral decubitus position. The initial three milliliters were used for routine cell counts and clinical chemistry, and the following six milliliters were immediately frozen at the bedside in 12 aliquots of 500 µl each. Frozen CSF samples were stored at - 80 °C until biochemical analyses.

### Enzyme-linked immunosorbent assay (ELISA)

For measuring CSF concentrations of cystatin C, a modified version of the Olafson et al. sandwich ELISA protocol was used (Olafson et al., Scand. J. Clin. Lab. Invest., 48, 573 - 582, 1988, which is hereby incorporated by reference). In brief, 50 µl of 1:500 dilutions of CSF samples or a dilution curve of purified human cystatin C (Calbiochem) were incubated in 96-well plates (Nunc Maxisorb) that were previously coated with a rabbit polyclonal anti-human cystatin C antiserum (DAKO, Germany) and blocked with 3% BSA, 0.05% Tween 20 in PBS. The biotinylated mouse monoclonal antibody HCC3 (provided by Dr. Magnus Abrahamson) against human cystatin C was used as a secondary antibody, it was detected by streptavidin conjugated with horseradish peroxidase (Amersham) and O-phenylenediamine (Sigma) as a chromagen read at 490 nm in an ELISA reader (Biorad). Diluted CSF samples were randomly distributed on the plates, assayed blinded towards diagnosis, and triplicates of each sample were measured on two separate occasions. The sensitivity of this assay was 0.5 ng/ml, it was linear over range of 1 to 200 ng/ml (r=0.998), and the coefficient of variance was less than 5% in all cases.

### Western blotting and densitometry

A Keyhole limpet hemosiderin-conjugated peptide of SEQ ID No. 1 (EGDPEAQRRVSKNSK) was used to immunize rabbits with to generate the antiserum R9672. The antiserum was affinity-purified after ammonium sulfate precipitation by binding to the above peptide conjugated to NHS-activated sepharose (HiTrap, Pharmacia). In CSF, this antiserum specifically detected the monomer form of cystatin C that migrated on 2D gels at a pH of 8.75, with a molecular mass of 13.3 kDa, the identical protein was detected by a commercial anti-human cystatin C antibody (Amava, Switzerland). The signal generated by either antibody was preadsorbable by preincubation of the antiserum with puried cystatin C, and densitometric measurements of signal intensities generated with human CSF samples correlated significantly (r = 0.952, P < 0.003). Together, the characterization of R9672 revealed that it specifically reacted in CSF with cystatin C, despite the fact that it was raised against, and detects in brain protein extracts, the 21 kDa presenilin 1 C-terminal fragment.

For the determination of monomer forms of cystatin C, 35 µl CSF were centrifuged and superfusate media were lyophilized by brief vacuum centrifugation, resuspended in 15 µl water and 15 µl 2X reducing SDS-gel loading buffer and heated to 37 °C for 10 minutes. 25 µl of the CSF protein extracts were loaded on each lane of 16% linear SDS polyacrylamide gels, separated by electrophoresis, electrotransferred to PVDF membranes (Immobilon), and probed with affinity-purified R9672. Anti-rabbit IgG conjugated to horseradish peroxidase (Amersham), as used as secondary antibody detected by chemiluminescence with preflashed (linear) X-ray films (Kodak). At the 1:10,000 dilution used for the anti-rabbit secondary antibody there was no detectable cross-reaction with human IgG derivatives present in the CSF protein extracts. A standard was prepared from a large volume of CSF obtained from a patient with normal pressure hydrocephalus, processed identically and splitted in 50 µl aliquots that were thawed only once before each gel run. Two standard samples were included on each gel; they were always processed identically and in parallel with the test samples. Samples were measured three times on three separate occasions. Immunoreactive bands were digitized by a flat bed scanner, and the optical densities were analyzed by the "NIH image" software package and normalized to the average signal generated by the standards on each blot. Measurements were always done in the linear range of the assay.

### Protein purification and peptide sequencing

Human CSF obtained by lumbar puncture was dialyzed against 10 mM Tris pH 7.8, loaded onto an FPLC Mono Q HR5/5 column (Pharmacia), and proteins were eluted with a stepwise gradient of 0 to 1 M NaCl in 10 mM Tris pH 7.8. Fractions were tested for immunoreactivity with R9672 by Western blotting. R9672-positive fractions eluted at 100 mM NaCl; they were separated by SDS-polyacrylamide gel electrophoresis, electroblotted onto immobilon-P^{SQ} membranes and stained with coomassie blue. The R9672-immunoreactive 13.3 kDa protein was subjected to microsequencing by Edman degradation on an automated Applied BioSystems 476A protein sequencer.

### Statistics

Statistical analysis was performed using methods known in the art. Such methods are described in detail in, for example, Snedecor G.W. and Cochran W.G., Statistical methods, 8th ed., lowa State University Press, Ames lowa 1989.

### Results

### Elevated CSF levels of total cystatin C in sporadic AD

CSF samples were taken from the above mentioned 56 sporadic AD patients, 38 patients with various non-AD neurological diseases (DDC), and 17 normal healthy control subjects (NC). By using ELISA, significantly higher CSF levels of cystatin C were found in AD patients than in either normal control subjects or patients with other non-AD neurological and psychiatric disorders as shown in Table 1. The non-AD neurological controls were not statistically different from the healthy controls (ELISA P = 0.177, Western P = 0.222). Samples for ELISA and Western blotting and densitometry measurements were derived from identical patients except for 9 non-AD neurological controls that were available for Western blotting only. Groups were compared by ANOVA and Scheffé-tests. Further, CSF levels of cystatin C in patients with non-AD neurological and psychiatric diseases did not differ statistically from our normal control subjects, and from normal CSF levels reported in the literature (Löfberg et al., J. Neurol. 223, 159 - 170, 1980; Grubb, New Engl. J. Med. 311, 1547 - 1549, 1984; Grubb et al., Scand. J. Clin. Lab. Invest. 45, Suppl. 177, 7 - 13, 1985) as shown in Table 2. Importantly, CSF levels of cystatin C in patients with non-AD neurodegenerative diseases, or with inflammatory CNS diseases were significantly lower than in AD patients and did not differ from normal controls. These data indicate that changes in cystatin C levels are not a non-specific response to inflammation or neurodegeneration, and argue in favor of a specific role of cystatin C in the pathophysiologie in AD. Receiver-operated characteristics analyses revealed that a cut-off value of 1.91 rel. O.D. for the monomer form of cystatin C was associated with a specificity of 94 % and a sensitivity of 73 % for the diagnosis of Alzheimer's disease.

### Elevated CSF levels of the monomer form of cystatin C in AD

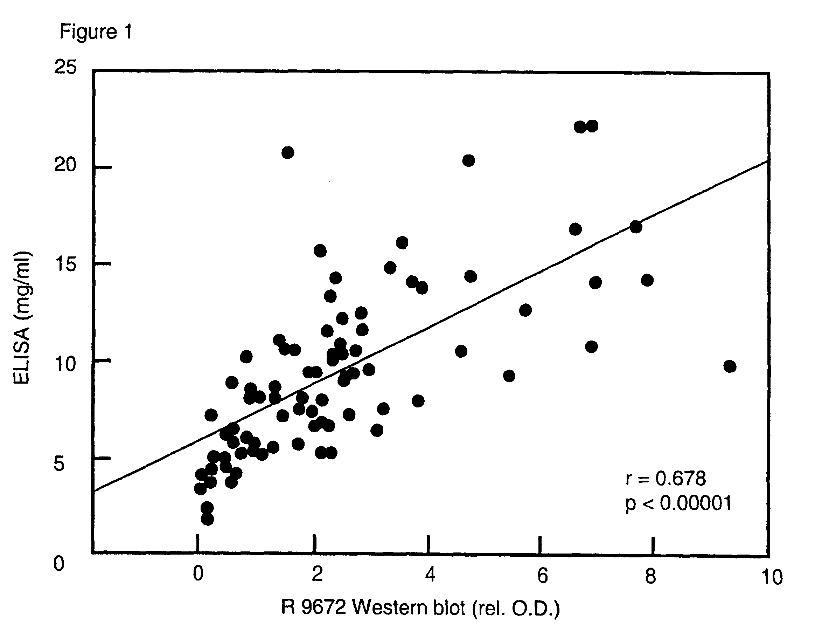

As mentioned above, cystatin C can form SDS-stable, biologically inactive dimers and oligomers. These are detected by the antibodies used for the ELISA. In order to measure CSF levels of the monomeric form of cystatin C, a quantitative Western blotting assay was used and the amount of monomer cystatin C was determined by densitometry of the 13.3 kDa immunoreactive monomer. Data from the Western blotting assay correlated with ELISA data obtained from identical samples as shown in figure 1. Densitometric analyses revealed 3.6-fold higher concentrations in AD patients as compared to normal controls (P = 0.0012), and two-fold higher concentrations in AD patients as compared to the non-AD neurological disease group (P = 0.0059) (Table 1). As with the ELISA data, mean values for the non-AD neurological and the normal control groups did not differ. Although the results from the two assay methods were similar, the differences in group means were larger in the Western blotting assay.

The mean ages of the control group were significantly less than the mean age of the AD group. To test whether age was the determinant factor for elevated levels of the cystatin C monomer in AD, the two control groups were combined to obtain sufficient numbers of elderly patients without AD. An analysis stratifying by decade was performed, using two-way analysis of variance. This analysis also showed a significant difference between the diagnoses (P = 0.04), as did an analysis of covariance that corrected for age as a continuous covariate (P = 0.02). There was a subtle but significant correlation between age and CSF levels of cystatin C monomers for non-AD individuals (r = 0.260, P = 0.048), but not in the AD group. Together, these analyses indicate that diagnosis, rather than age, accounted for the increased CSF levels of cystatin C in AD patients.

CSF levels of the cystatin C monomer within the AD group did not vary in association with ApoE genotype (Table 6), nor did they correlate significantly with measures of dementia severity, age of dementia onset, duration of illness, nor gender. In addition, there was a higher variance (P < 0.01; F-test) in ApoE ε3 homozygous individuals with AD, caused by a subgroup of *ApoE* ε3/3 carriers with high CSF levels of cystatin C monomers. Together, these data suggest that CSF levels of cystatin C are independent of *ApoE* genotype.

Significantly more AD patients with mild to moderate stages of dementia had higher than normal (cystatin C > 2 rel. OD) CSF levels of the cystatin C monomer than severely demented patients (P < 0.01, χ² test). Comparisons of mildly demented AD patients (MMSE 18 - 30) to moderately to severely impaired patients (MMSE < 18) by χ² test also showed that more patients (P < 0.025) with mild stages of dementia had high levels of cystatin C as compared to the more severely impaired patients. This finding indicates that the metabolic abnormality leading to abnormally high CSF levels of cystatin C occurs early in the course of the disease. It raises the possibility of using CSF measurements of cystatin C in the early diagnosis of AD.

### PS1 loop antibodies crossreact with cystatin C in CSF

Even though cystatin C and PS1 do not have sequence homologies, the affinity-purified antibody R9672 strongly reacted with both the PS1 C-terminal fragment (PS1-CTF) in protein extracts prepared from human brain or transfected cells and with cystatin C derived from either human CSF or human urine. The binding of R9672 to both proteins was preadsorbable either by the PS1-derived peptide used for immunizations or by purified, denatured cystatin C. To exclude the possibility that cystatin C and a theoretical PS1-derived fragment co-migrated at 13.3 kDa on SDS gels, a two-dimensional gel electrophoresis was used to separate CSF proteins by pl in the first dimension, and by molecular mass in the second dimension. The separated proteins were transferred to Immobilon membrane filters and probed with either R9672 or with commercially available cystatin C antibodies. Both antibodies detected the same protein that migrated with 13.3 kDa at a pH of 8.75. These data matched exactly the theoretical molecular mass and pl of the 120 residue mature cystatin C after removal of the signal peptide. There were no other immunoreactive proteins detected by either antibody on this 2D Western blotting assay. In particular, there was no immunoreactive protein seen with R9672 at other pH values including 4.26, the pl of a 13.3 kDa theoretical N-terminal PS1-CTF derivative generated by presenilinase cleavage.

The R9672 Western blotting assay was used to purify the 13.3 kDa protein from human CSF by combined ion exchange chromatography and SDS-polyacrylamide electrophoresis to determine its N-terminus by automated Edman degradation sequencing. Sequence analysis unequivocally revealed SSPGXPPRLVGGPMXA (SEQ ID NO. 2), corresponding with 100 % identity to the N-terminus of cystatin C without the signal peptide. There were no additional sequences detectable in this preparation.

### EXAMPLE 2

### Patient characteristics in the genetic study

145 controls (121 cognitively normal controls and 24 cognitively normal patients with non-AD neurological diseases) and 69 AD patients were included in the genetic association study. In 55 from these, CSF samples were available for the determination of cystatin C (see description of example 1).

### Amplification and genotyping of CST3, A2M and ApoE

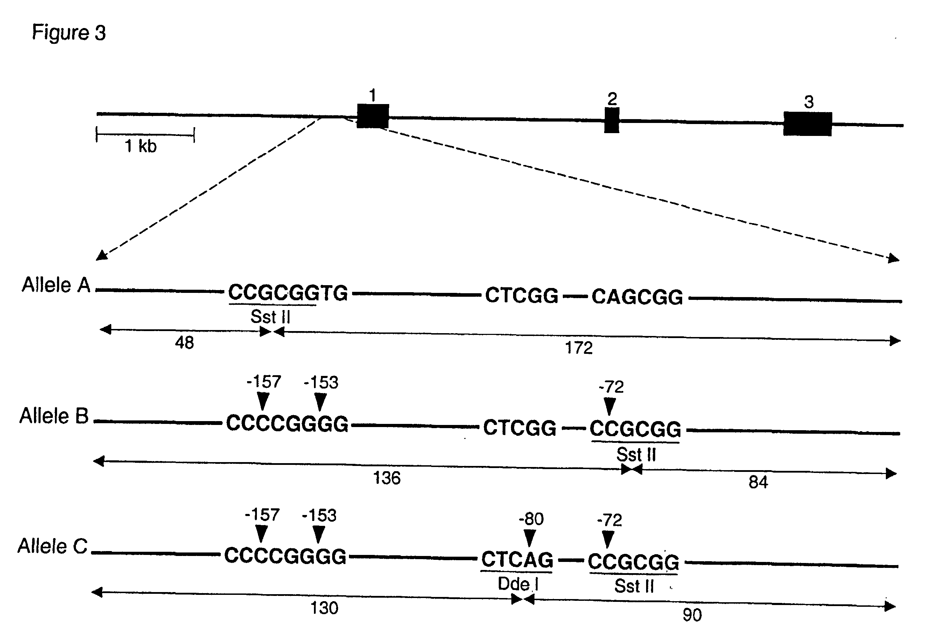

By use of polymerase chain reaction (PCR), a 318 bp segment that comprises parts of the putative promoter region of the cystatin C gene as well as the coding sequence of exon 1 (see Fig. 3), has been amplified (see Fig. 3 regarding mutations in the promoter region; an Ala/Thr exchange in the coding region of the human cystatin C gene has been detected as a *Sst* II polymorphism by Balbin et al., Hum. Genet. 92: 206 - 207, 1993). The primers used had sequences 5'-TGGGAGGGACGAGGCGTTCC-3' (SEQ ID NO. 3) and 5'-TCCATGGGGCCTCCCACCAG-3' (SEQ ID NO. 4). Amplification was carried out in a thermocycler under standard conditions without additives. PCR products were digested with *Sst* II or its isoschizomer *Sac* II for 1 hr at 37 °C and separated by 4 % agarose gel electrophoresis. *CST-3* B alleles derived restriction fragments are apparent in this system with 127 and 191 bp in length. *A2M* and *ApoE* were genotyped according to standard protocols.

### Results

### Genetic association of CST3 with AD

The human cystatin C gene (*CST3* ) was analyzed in an exploratory case-control study of 69 patients with a clinical diagnosis of AD and 145 controls without AD. Analysis of the *Sst* II polymorphism in the 5' flanking region of *CST3* (*CST3* B) revealed that it is associated with increased risk for AD. The frequency of the CST3 B allele was 0.254 in the AD patients as compared to 0.169 in the controls (P = 0.036). The frequency of the *CST3* B/B genotype in AD was 0.101 as compared to 0.025 in controls (P = 0.0141, Fisher's exact test) as shown in Table 3. The presence of two CST3 B alleles was associated with an odds ratio of 5.34 (95% Cl = {1.34, 21.35}, P = 0.009, Mantel-Haenszel test). By comparison, the odds ratio for *ApoE* ε4 carriers in the same data set was 3.43 (95% Cl = {1.75, 6.77}, P = 0.0003, Mantel-Haenszel test) as shown in Table 4. In an age-corrected data set of 96 controls above the age of 61 years and 70 AD patients, the frequency of *CST3* B/B was 0.0104 in the controls compared to 0.101 in AD (P = 0.00976, Fisher's exact test) as shown in Table 3. In this age-matched case-control design, the *CST3* B/B genotype was associated with an odds ratio of 10.7 (95% Cl = {1.28, 89.3}, P = 0.0074, Mantel-Haenszel test) as shown in Table 4.

The combination of either a B/B genotype and/or the presence of an *ApoE* ε4 allele resulted in an odds ratio of 4.54 (95% Cl = {2.25, 9.15}, p < 0.00001), which is higher than *ApoE* ε4 alone, but lower than *CST3* B/B alone. This difference reflects the known high number of *ApoE* ε4 positive individuals in the control group. No subjects with combined *CST3* B/B and *ApoE* ε4/4 genotypes were observed, and all control subjects with *CST3* B/B had *ApoE* ε3/3 genotypes. The *A2M* G/G genotype was not associated with AD in the present data set which is not necessarily in contrast with the reported association of *A2M* G/G because much larger sample sizes are required to detect it. The *CST3* B/B genotype was not associated with decreased ages of disease onset, analyzed either by ANOVA or by Kaplan-Meier analysis of age of onset of AD patients grouped according to CST3 genotype.

### Elevated CSF levels of cystatin C in CST3 B/B carriers

To determine whether the *CST3* genotype influences CSF levels of cystatin C, 55 individuals from whom ELISA data of CSF levels of cystatin C were available were grouped according to *CST3* genotype (*CST3* A/A: n=31; *CST3* A/B: n = 19; *CST3* B/B: n=5). CSF levels of cystatin C in B/B carriers were significantly higher (P = 0.032, ANOVA) as compared to these in *CST3* A carriers (Table 5).

### EXAMPLE 3

All CSF samples used in Examples 1 and 2 were aliquoted and frozen on dry ice immediately upon withdrawal at the bed side, stored at - 80 °C, and thawed only immediately prior to ELISAs or Western blotting assays.

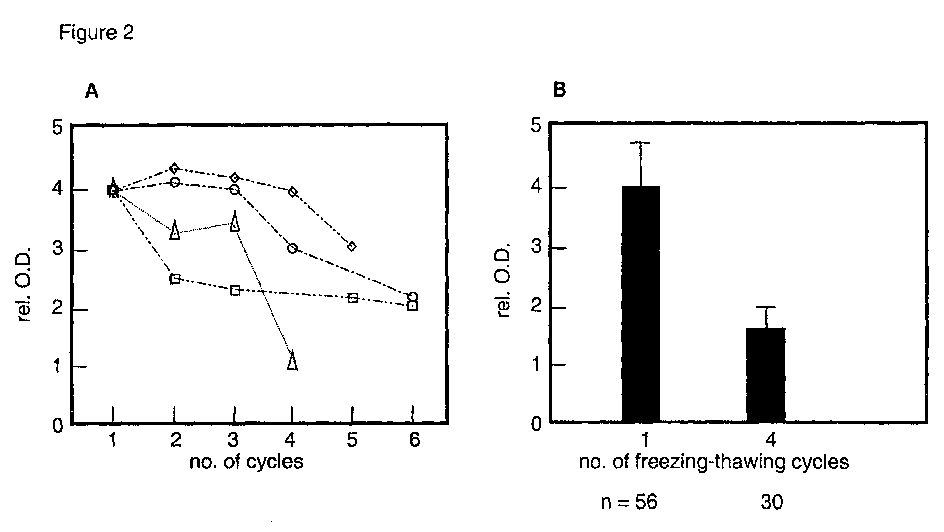

Figure 2 depicts the influence of repeated thawing and freezing CSF samples on their cystatin C content. Figure 2a shows the inter-individual variability of the sensitivity to the number of thaw-freeze cycles. In some samples, levels of the cystatin C monomer decreased 50% after two cycles, whereas in others it remained stable. After four or five freezing and thawing cycles, however, all samples consistently had significantly lower levels of the monomeric form of cystatin C. Figure 2b shows cystatin C levels after 1 and 4 freezing-thawing cycles, respectively.

CSF levels measured by ELISA were somewhat more stable towards repeated thawing and freezing, despite a clear tendency towards lower values after several cycles which might be due to the fact that the antibodies used in ELISA also detected dimers and oligomers. Consistent with this observation, low levels of cystatin C monomers were found in CSF samples derived from a source where the samples had been thawed and frozen several times prior to Western blotting. Prolonged un-interrupted freezing at - 80 °C between 1 and 114 months had no effect on CSF levels of cystatin C.

## Claims

1. A method for diagnosing or prognosing Alzheimer's disease in a subject, or determining whether a subject is at increased risk of developing Alzheimer's disease, comprising;
determining a level, or an activity, or both said level and said activity, of a transcription product and/or a translation product of (i) a cystatin C gene or (ii) a polymorphic variant of a cystatin C gene in a sample of cerebrospinal fluid from said subject;
and
comparing said level, or said activity, or both said level and said activity, of said transcription product and/or said translation product to a reference value representing a known disease or health status, whereby an increase of a level or a varied activity of a translation product of (I) a cystatin C gene or (ii) a polymorphic variant of a cystatin C gene in said cerebrospinal fluid from said subject relative to a reference value representing a known health status indicates a diagnosis, or prognosis, or increased risk of Alzheimer's disease in said subject.

2. A method of monitoring the progression of Alzheimer's disease in a subject, comprising:
determining a level, or an activity, or both said level and said activity, of a transcription product and/or a translation product of (i) a cystatin C gene or (ii) a polymorphic variant of a cystatin C gene in a sample of cerebrospinal fluid from said subject;
and
comparing said level, or said activity, or both said level and said activity, of said transcription product and/or said translation product to a reference value representing a known disease or health status, thereby monitoring the progression of Alzheimer's disease in said subject.

3. A method of evaluating a treatment for Alzheimer's disease, comprising:
determining a level, or an activity, or both said level and said activity, of a transcription product and/or a translation product of (i) a cystatin C gene or (ii) a polymorphic variant of a cystatin C gene in a cerebrospinal fluid sample obtained from a subject being treated for Alzheimer's disease;
and
comparing said level, or said activity, or both said level and said activity, of said transcription product and/or said translation product to a reference value representing a known disease or health status,
thereby evaluating said treatment for Alzheimer's disease.

4. The method according to one of claims 1 to 3, wherein said subject is a human.

5. The method according to one of daims 1 to 4, wherein said translation product of (i) a cystatin C gene or (ii) a polymorphic variant of a cystatin C gene is determined in its monomer form.

6. The method according to one of claims 1 to 5, wherein said translation product and/or said transcription product is detected using an immunoassay, an enzyme activity assay and/or a binding assay.

7. The method according to one of claims 1 to 6, wherein said reference value is that of a level, or an activity, or both said level and said activity, of a transcription product and/or a translation product of (i) a cystatin C gene or (ii) a polymorphic variant of a cystatin C gene in a sample from a subject not suffering from said Alzhelmer's disease.

8. The method according to one of claims 1 to 7, further comprising comparing a level, or an activity, or both said level and said activity, of a transcription product and/or a translation product of (i) a cystatin C gene or (ii) a polymorphic variant of a .cystatin C gene, in said sample with a level, an activity, or both said level and said activity, of a transcription product and/or a translation product of (i) a cystatin C gene or (ii) a polymorphic variant of a cystatin C gene in a series of samples taken from said subject over a period of time.

9. The method according to claim 8, wherein said subject receives a treatment prior to one or more sample gatherings.

10. The method of claim 9, wherein said level, or said activity, or both said level and said activity, in said samples is determined, before and after said treatment of said subject.

11. A method of diagnosing or prognosing Alzheimer's disease in a subject, or determining whether a subject is at increased risk of developing Alzheimer's disease comprising:
determining a presence or absence of a polymorphism in a cystatin C gene in a sample from said subject,
thereby diagnosing or prognosing Alzheimer's disease in said subject, or determining whether said subject is at increased risk of developing Alzheimer's disease.

12. The method of claim 11, wherein the polymorphism is not in leucin 68 codon of a human cystatin C gene leading to a loss of *Alu* I restriction site.

13. The method of daim 11 and/or 12, wherein the presence or absence of at least one B allele is determined.

14. The method of claim 13, wherein the presence of at least one B allele, in particular the presence of the B/B genotype, indicates said subject is at increased risk of developing Alzheimer's disease or indicates a diagnosis or prognosis of Alzheimer's disease.

15. The method of at least one of claims 11 to 14, further comprising:
determining a level, or an activity, or both said level and said activity, of a transcription product and/or a translation product of (i) a cystatin C gene or (ii) a polymorphic variant of a cystatin C gene in a sample from said subject;
and
comparing said level, or said activity, or both said level and said activity, of said transcription product and/or said translation product to a reference value representing a known disease or health status.

16. A kit for the diagnosis or prognosis, or determination of increased risk of developing Alzheimer's disease in a subject, said kit comprising:
reagents that selectively detect the presence or absence of the β allele of the cystatin C gene.

17. The kit according to claim 16, further comprising
reagents that selectively detect a transcription product of (i) a cystatin C gene or (ii) a polymorphic variant of a cystatin C gene and/or reagents that selectively detect a translation product of (i) a cystatin C gene or (ii) a polymorphic variant of a cystatin C gene.

18. The kit according to claim 16 and/or 17, wherein the polymorphism is not in leucin 68 codon of a human cystatin C gene leading to a loss of *Alu* I restriction site.

19. The kit according to one of claims 16 to 18, further comprising reagents to assess a function or dysfunction of said subject's kidneys.

20. The kit according to one of claims 16 to 19, wherein said translation product of (i) a cystatin C gene or (ii) a polymorphic variant of a cystatin C gene is determined in its monomer form.

## Patentansprüche

1. Verfahren zum Diagnostizieren oder Prognostizieren von Alzheimer-Krankheit bei einem Patienten oder zum Bestimmen, ob ein Patient ein erhöhtes Risiko hat, eine Alzheimer-Krankheit zu entwickeln, umfassend:
Bestimmen einer Konzentration oder einer Aktivität oder sowohl einer Konzentration als auch einer Aktivität eines Transcriptionsprodukts und/oder eines Translationsprodukts von (i) einem Cystatin-C-Gen oder (ii) einer polymorphen Variante eines Cystatin-C-Gens in einer Liquorprobe von dem Patienten; und
Vergleichen der Konzentration oder der Aktivität oder sowohl der Konzentration als auch der Aktivität des Transcriptionsprodukts und/oder Translationsprodukts mit einem Referenzwert, der einen bekannten Krankheitsoder Gesundheitszustand darstellt; wobei eine Erhöhung einer Konzentration oder eine variierte Aktivität eines Translationsprodukts von (i) einem Cystatin-C-Gen oder (ii) einer polymorphen Variante eines Cystatin-C-Gens in dem Liquor von dem Patienten relativ zu einem Referenzwert, der einen bekannten Gesundheitszustand darstellt, eine Diagnose oder Prognose oder ein erhöhtes Risiko von Alzheimer-Krankheit bei dem Patienten anzeigt.

2. Verfahren zur Überwachung des Fortschritts von Alzheimer-Krankheit bei einem Patienten, umfassend:
Bestimmen einer Konzentration oder einer Aktivität oder sowohl einer Konzentration als auch einer Aktivität eines Transcriptionsprodukts und/oder eines Translationsprodukts von (i) einem Cystatin-C-Gen oder (ii) einer polymorphen Variante eines Cystatin-C-Gens in einer Liquorprobe von dem Patienten; und
Vergleichen der Konzentration oder der Aktivität oder sowohl der Konzentration als auch der Aktivität des Transcriptionsprodukts und/oder Translationsprodukts mit einem Referenzwert, der einen bekannten Krankheitsoder Gesundheitszustand darstellt;
wodurch der Fortschritt der Alzheimer-Krankheit bei dem Patienten überwacht wird.

3. Verfahren zur Bewertung einer Behandlung auf Alzheimer-Krankheit, umfassend:
Bestimmen einer Konzentration oder einer Aktivität oder sowohl einer Konzentration als auch einer Aktivität eines Transcriptionsprodukts und/oder eines Translationsprodukts von (i) einem Cystatin-C-Gen oder (ii) einer polymorphen Variante eines Cystatin-C-Gens in einer Liquorprobe, die von einem Patienten erhalten wurde, der auf Alzheimer-Krankheit behandelt wird; und
Vergleichen der Konzentration oder der Aktivität oder sowohl der Konzentration als auch der Aktivität des Transcriptionsprodukts und/oder Translationsprodukts mit einem Referenzwert, der einen bekannten Krankheitsoder Gesundheitszustand darstellt;
wodurch die Behandlung auf Alzheimer-Krankheit bewertet wird.

4. Verfahren gemäß einem der Ansprüche 1 bis 3, wobei der Patient ein Mensch ist.

5. Verfahren gemäß einem der Ansprüche 1 bis 4, wobei das Translationsprodukt von (i) einem Cystatin-C-Gen oder (ii) einer polymorphen Variante eines Cystatin-C-Gens in seiner monomeren Form bestimmt wird.

6. Verfahren gemäß einem der Ansprüche 1 bis 5, wobei das Translationsprodukt und/oder Transcriptionsprodukt unter Verwendung eines Immunoassays, eines Enzymaktivitätsassays und/oder eines Bindungsassays nachgewiesen wird.

7. Verfahren gemäß einem der Ansprüche 1 bis 6, wobei der Referenzwert der Referenzwert einer Konzentration oder einer Aktivität oder sowohl einer Konzentration als auch einer Aktivität eines Transcriptionsprodukts und/oder eines Translationsprodukts von (i) einem Cystatin-C-Gen oder (ii) einer polymorphen Variante eines Cystatin-C-Gens in einer Probe von einem Patienten, der nicht an Alzheimer-Krankheit leidet, ist.

8. Verfahren gemäß einem der Ansprüche 1 bis 7, das weiterhin folgendes umfasst: Vergleichen einer Konzentration oder einer Aktivität oder sowohl einer Konzentration als auch einer Aktivität eines Transcriptionsprodukts und/oder eines Translationsprodukts von (i) einem Cystatin-C-Gen oder (ii) einer polymorphen Variante eines Cystatin-C-Gens in der Probe mit einer Konzentration oder einer Aktivität oder sowohl einer Konzentration als auch einer Aktivität eines Transcriptionsprodukts und/oder eines Translationsprodukts von (i) einem Cystatin-C-Gen oder (ii) einer polymorphen Variante eines Cystatin-C-Gens in einer Reihe von Proben, die über einen Zeitraum hinweg von dem Patienten entnommen wurden.

9. Verfahren gemäß Anspruch 8, wobei der Patient vor einer oder mehreren der Probeentnahmen eine Behandlung erhält.

10. Verfahren gemäß Anspruch 9, wobei die Konzentration oder die Aktivität oder sowohl die Konzentration als auch die Aktivität in den Proben vor und nach der Behandlung des Patienten bestimmt wird.

11. Verfahren zum Diagnostizieren oder Prognostizieren von Alzheimer-Krankheit bei einem Patienten oder zum Bestimmen, ob ein Patient ein erhöhtes Risiko hat, eine Alzheimer-Krankheit zu entwickeln, umfassend:
Bestimmen einer Anwesenheit oder Abwesenheit eines Polymorphismus in einem Cystatin-C-Gen in einer Probe von dem Patienten;
wodurch die Alzheimer-Kränkheit bei dem Patienten diagnostiziert oder prognostiziert wird oder bestimmt wird, ob der Patient ein erhöhtes Risiko hat, Alzheimer-Krankheit zu entwickeln.

12. Verfahren gemäß Anspruch 11, wobei sich der Polymorphismus nicht im Leucin-68-Codon eines Human-Cystatin-C-Gens befindet, der zu einem Verlust einer AluI-Restriktionsstelle führt.

13. Verfahren gemäß Anspruch 11 und/oder 12, wobei die Anwesenheit oder Abwesenheit wenigstens eines B-Allels bestimmt wird.

14. Verfahren gemäß Anspruch 13, wobei die Anwesenheit wenigstens eines B-Allels, insbesondere die Anwesenheit des B/B-Genotyps, anzeigt, dass der Patient ein erhöhtes Risiko hat, Alzheimer-Krankheit zu entwickeln, oder eine Diagnose oder Prognose von Alzheimer-Krankheit anzeigt.

15. Verfahren gemäß wenigstens einem der Ansprüche 11 bis 14, das weiterhin folgendes umfasst:
Bestimmen einer Konzentration oder einer Aktivität oder sowohl einer Konzentration als auch einer Aktivität eines Transcriptionsprodukts und/oder eines Translationsprodukts von (i) einem Cystatin-C-Gen oder (ii) einer polymorphen Variante eines Cystatin-C-Gens in einer Probe von dem Patienten; und
Vergleichen der Konzentration oder der Aktivität oder sowohl der Konzentration als auch der Aktivität des Transcriptionsprodukts und/oder Translationsprodukts mit einem Referenzwert, der einen bekannten Krankheitsoder Gesundheitszustand darstellt.

16. Kit für die Diagnose oder Prognose von Alzheimer-Krankheit oder zur Bestimmung eines erhöhten Risikos, Alzheimer-Krankheit zu entwickeln, bei einem Patienten, wobei der Kit Folgendes umfasst: Reagentien, die selektiv die Anwesenheit oder Abwesenheit des B-Allels des Cystatin-C-Gens nachweisen.

17. Kit gemäß Anspruch 16, der weiterhin Folgendes umfasst:
Reagentien, die selektiv ein Transcriptionsprodukt von (i) einem Cystatin-C-Gen oder (ii) einer polymorphen Variante eines Cystatin-C-Gens nachweisen; und/oder
Reagentien, die selektiv ein Translationsprodukt von (i) einem Cystatin-C-Gen oder (ii) einer polymorphen Variante eines Cystatin-C-Gens nachweisen.

18. Kit gemäß Anspruch 16 und/oder 17, wobei sich der Polymorphismus nicht im Leucin-68-Codon eines Human-Cystatin-C-Gens befindet, der zu einem Verlust einer AluI-Restriktionsstelle führt.

19. Kit gemäß einem der Ansprüche 16 bis 18, der weiterhin Reagentien zur Bewertung einer Funktion oder Dysfunktion der Nieren des Patienten umfasst.

20. Kit gemäß einem der Ansprüche 16 bis 19, wobei das Translationsprodukt von (i) einem Cystatin-C-Gen oder (ii) einer polymorphen Variante eines Cystatin-C-Gens in seiner monomeren Form bestimmt wird.

## Revendications

1. Une méthode pour diagnostiquer ou pronostiquer la maladie d'Alzheimer chez un sujet, ou déterminer si un sujet présente un risque accru de développer la maladie d'Alzheimer, comprenant :
la détermination d'un taux, ou d'une activité, ou à la fois dudit taux et de ladite activité, d'un produit de transcription et/ou d'un produit de traduction de (i) un gène de cystatine C ou (ii) un variant polymorphe d'un gène de cystatine C dans un échantillon de liquide céphalo-rachidien provenant dudit sujet ;
et
la comparaison dudit taux, ou de ladite activité, ou à la fois dudit taux et de ladite activité, dudit produit de transcription et/ou dudit produit de traduction avec une valeur de référence représentant un état de maladie ou de santé connu, en sorte qu'une augmentation d'un taux ou une activité modifiée d'un produit de traduction de (i) un gène de cystatine C ou (ii) un variant polymorphe d'un gène de cystatine C contenu dans ledit liquide céphalo-rachidien provenant dudit sujet par rapport à une valeur de référence représentant un état de santé connu indique un diagnostic ou un pronostic ou un risque accru de maladie d'Alzheimer chez ledit sujet.

2. Une méthode pour surveiller la progression de la maladie d'Alzheimer chez un sujet, comprenant :
la détermination d'un taux, ou d'une activité, ou à la fois dudit taux et de ladite activité, d'un produit de transcription et/ou d'un produit de traduction de (i) un gène de cystatine C ou (ii) un variant polymorphe d'un gène de cystatine C dans un échantillon de liquide céphalo-rachidien provenant dudit sujet ;
et
la comparaison dudit taux, ou de ladite activité, ou à la fois dudit taux et de ladite activité, dudit produit de transcription et/ou dudit produit de traduction avec une valeur de référence représentant un état de maladie ou de santé connu, pour surveiller ainsi la progression de la maladie d'Alzheimer chez ledit sujet.

3. Une méthode pour évaluer un traitement de la maladie d'Alzheimer, comprenant :
la détermination d'un taux, ou d'une activité, ou à la fois dudit taux et de ladite activité, d'un produit de transcription et/ou d'un produit de traduction de (i) un gène de cystatine C ou (ii) un variant polymorphe d'un gène de cystatine C dans un échantillon de liquide céphalo-rachidien provenant d'un sujet qui est traité pour la maladie d'Alzheimer ;
et
la comparaison dudit taux, ou de ladite activité, ou à la fois dudit taux et de ladite activité, dudit produit de transcription et/ou dudit produit de traduction avec une valeur de référence représentant un état de maladie ou de santé connu, pour évaluer ainsi ledit traitement de la maladie d'Alzheimer.

4. La méthode selon l'une des revendications 1 à 3, dans laquelle ledit sujet est un être humain.

5. La méthode selon l'une des revendications 1 à 4, dans laquelle ledit produit de traduction de (i) un gène de cystatine C ou (ii) un variant polymorphe d'un gène de cystatine C est déterminé sous sa forme monomère.

6. La méthode selon l'une des revendications 1 à 5, dans laquelle ledit produit de traduction et/ou ledit produit de transcription sont détectés en utilisant un essai immunologique, un essai d'activité enzymatique et/ou un essai par liaison.

7. La méthode selon l'une des revendications 1 à 6, dans laquelle ladite valeur de référence est celle d'un taux, ou d'une activité, ou à la fois dudit taux et de ladite activité, d'un produit de transcription et/ou d'un produit de traduction de (i) un gène de cystatine C ou (ii) d'un variant polymorphe d'un gène de cystatine C dans un échantillon provenant d'un sujet ne souffrant pas de ladite maladie d'Alzheimer.

8. La méthode selon l'une des revendications 1 à 7, comprenant de plus la comparaison d'un taux, ou d'une activité, ou à la fois dudit taux et de ladite activité, d'un produit de transcription et/ou d'un produit de traduction de (i) un gène de cystatine C ou (ii) un variant polymorphe d'un gène de cystatine C dans ledit échantillon avec un taux, une activité, ou à la fois ledit taux et ladite activité, d'un produit de transcription et/ou d'un produit de traduction de (i) un gène de cystatine C ou (ii) un variant polymorphe d'un gène de cystatine C dans une série d'échantillons prélevés audit sujet durant une certaine période de temps.

9. La méthode selon la revendication 8, dans laquelle ledit sujet reçoit un traitement avant une ou plusieurs collectes d'échantillons.

10. La méthode de la revendication 9, dans laquelle ledit taux, ou ladite activité, ou à la fois ledit taux et ladite activité, dans lesdits échantillons sont déterminés avant et après ledit traitement dudit sujet.

11. Une méthode pour diagnostiquer ou pronostiquer la maladie d'Alzheimer chez un sujet, ou déterminer si un sujet présente un risque accru de développer la maladie d'Alzheimer, comprenant :
la détermination d'une présence ou absence d'un polymorphisme dans un gène de cystatine C dans un échantillon provenant dudit sujet,
pour diagnostiquer ou pronostiquer ainsi la maladie d'Alzheimer chez ledit sujet, ou déterminer ainsi si ledit sujet présente un risque accru de développer la maladie d'Alzheimer.

12. La méthode de la revendication 11, dans laquelle le polymorphisme ne se trouve pas dans le codon 68 de la leucine d'un gène de cystatine C humain en conduisant à une perte de site de restriction *Alu*I.

13. La méthode de la revendication 11 et/ou 12, dans laquelle la présence ou l'absence d'au moins un allèle B est déterminée.

14. La méthode de la revendication 13, dans laquelle la présence d'au moins un allèle B, en particulier la présence du génotype B/B, indique que ledit sujet présente un risque accru de développer la maladie d'Alzheimer ou indique un diagnostic ou un pronostic de la maladie d'Alzheimer.

15. La méthode d'au moins l'une des revendications 11 à 14, comprenant de plus :
la détermination d'un taux, ou d'une activité, ou à la fois dudit taux et de ladite activité, d'un produit de transcription et/ou d'un produit de traduction de (i) un gène de cystatine C ou (ii) un variant polymorphe d'un gène de cystatine C dans un échantillon provenant dudit sujet ;
et
la comparaison dudit taux, ou de ladite activité, ou à la fois dudit taux et de ladite activité, dudit produit de transcription et/ou dudit produit de traduction avec une valeur de référence représentant un état de maladie ou de santé connu.

16. Un nécessaire pour le diagnostic ou le pronostic, ou la détermination d'un risque accru de développer la maladie d'Alzheimer chez un sujet, ledit nécessaire comprenant :
des réactifs qui détectent sélectivement la présence ou l'absence de l'allèle B du gène de cystatine C.

17. Le nécessaire selon la revendication 16, comprenant de plus des réactifs qui détectent sélectivement un produit de transcription de (i) un gène de cystatine C ou (ii) un variant polymorphe d'un gène de cystatine C et/ou
des réactifs qui détectent sélectivement un produit de traduction de (i) un gène de cystatine C ou (ii) un variant polymorphe d'un gène de cystatine C.

18. Le nécessaire selon la revendication 16 et/ou la revendication 17, dans lequel le polymorphisme ne se trouve pas dans le codon 68 de la leucine d'un gène de cystatine C humain en conduisant à une perte de site de restriction *Alu*I.

19. Le nécessaire selon l'une des revendications 16 à 18, comprenant de plus des réactifs pour apprécier une fonction ou dysfonction des reins dudit sujet.

20. Le nécessaire selon l'une des revendications 16 à 19, dans lequel ledit produit de traduction de (i) un gène de cystatine C ou (ii) un variant polymorphe d'un gène de cystatine C est déterminé sous sa forme monomère.
